(19) Europäisches Patentamt

European Patent Office

Office européen des brevets

(11) Veröffentlichungsnummer: **0 559 851 B1**

# (12) EUROPÄISCHE PATENTSCHRIFT

(45) Veröffentlichungstag der Patentschrift: **23.08.95**

(51) Int. Cl.⁶: **A61K 7/09**

(21) Anmeldenummer: **92917517.2**

(22) Anmeldetag: **19.08.92**

(86) Internationale Anmeldenummer:
**PCT/EP92/01888**

(87) Internationale Veröffentlichungsnummer:
**WO 93/05757 (01.04.93 93/09)**

---

(54) **FIXIERMITTEL UND VERFAHREN ZUR DAUERHAFTEN HAARVERFORMUNG.**

---

(30) Priorität: **26.09.91 DE 4131992**

(43) Veröffentlichungstag der Anmeldung:
**15.09.93 Patentblatt 93/37**

(45) Bekanntmachung des Hinweises auf die
Patenterteilung:
**23.08.95 Patentblatt 95/34**

(84) Benannte Vertragsstaaten:
**DE ES FR GB IT**

(56) Entgegenhaltungen:
**EP-A- 0 218 931**
**EP-A- 0 356 665**
**WO-A-90/00385**
**GB-A- 2 116 218**
**GB-A- 2 170 830**

**PATENT ABSTRACTS OF JAPAN vol. 9, no. 78 (C-274)6. April 1985**

(73) Patentinhaber: **Wella Aktiengesellschaft**
**Berliner Allee 65**
**D-64295 Darmstadt (DE)**

(72) Erfinder: **FRIEDERICH, Rainer**
**Heinrich-von-Kleist-Strasse 38**
**D-6093 Biebesheim (DE)**
Erfinder: **MARESCH, Gerhard**
**Winkelschneise 6**
**D-6100 Darmstadt (DE)**

---

**Beschreibung**

Die vorliegende Erfindung betrifft ein flüssiges, leicht angedicktes Mittel zur Durchführung der oxidativen Nachbehandlung bei der dauerhaften Haarverformung, welches eine Mischung aus einem Oxidationsmittel, einer amphoteren oberflächenaktiven Verbindung, einem kationischen Polymer sowie einer nichtionogenen oberflächenaktiven Verbindung enthält, sowie ein Verfahren zur dauerhaften Haarverformung unter Verwendung dieses Fixiermittels.

Bei der dauerhaften Haarverformung werden die Haare in der Regel zunächst mit einem Verformungsmittel auf der Basis einer keratinreduzierenden Mercaptoverbindung, welche eine Öffnung der Disulfidbrücken des Haarkeratins bewirkt, behandelt und sodann in die gewünschte Form gebracht. Als Verformungsmittel werden in der Regel keratinreduzierende Mercaptoverbindungen, wie zum Beispiel Salze oder Ester von Mercaptocarbonsäuren, verwendet. Anschließend wird das Haar mit Wasser gespült und sodann mit einem Fixiermittel oxidativ nachbehandelt. Hierbei werden die zuvor gespaltenen Disulfidbrücken in der neuen Form wieder verknüpft.

Durch die bei dieser Verfahrensweise auf das Haar einwirkenden chemischen und mechanischen Vorgänge wird die Haarqualität, insbesondere bezüglich Griff und Kämmbarkeit des Haares, verschlechtert und das Haar wird spröde und rauh.

Es wurde bereits versucht, durch Zusatz bestimmter pflegender Substanzen zu dem Verformungsmittel oder dem Fixiermittel, dem Haar einen glatten, angenehmen Griff zu verleihen sowie die Kämmbarkeit der Haare zu verbessern. Als besonders vorteilhaft hat sich hierbei die Verwendung von kationischen Verbindungen, beispielsweise kationischen Polymeren, in Kombination mit unterschiedlichen Tensiden sowie weiteren Zusätzen in Fixiermitteln erwiesen.

So beschreibt die GB-PS 2 170 830 die Verwendung saurer Fixiermittel auf der Basis von Wasserstoffperoxid, welche ein kationisches Polymer sowie ein amphoteres oder anionisches Tensid enthalten.

Desweiteren wird in unserer eigenen DE-OS 38 26 369 empfohlen, zur Durchführung der oxidativen Nachbehandlung bei der dauerhaften Haarverformung ein Mittel auf der Basis eines oxidierenden Wirkstoffes, eines kationischen Polymers und/oder eines kationischen Tensides sowie eines Salzes der 2-Pyrrolidon-5-carbonsäure zu verwenden, welches eine gute Konditionierung der Haare ermöglicht, mit Wasser leicht ausspülbar ist und ein gutes Verformungsergebnis gewährleistet.

Alle diese beschriebenen Fixiermittel weisen jedoch Nachteile auf. So führen diese Mittel, beispielsweise aufgrund einer ungenügenden Verdickung infolge übermäßigen Ablaufens vom Haar, zu Kopfhautkontakten und somit möglichen unangenehmen Hautreizungen oder aber infolge einer zu starken Verdickung zu einer unbefriedigenden Fixierung des dauerverformten Haares.

Langzeitverdickungen auf polymerer Basis besitzen zudem, ebenso wie viele Tensidzusätze, oft eine destabilisierenden Einfluß auf die zum Einsatz gelangenden Oxidationsmittel, so daß sich entweder anwendungstechnisch zwangsläufig schlechtere Umformungsergebnisse infolge eines reduzierten Oxidationsmittelgehaltes oder aber sogar gefährliche Deformationen beziehungsweise Bombagen der Verpackungen dieser Mittel ergeben.

Es bestand daher die Aufgabe, ein Mittel zur Durchführung der oxidativen Nachbehandlung bei der dauerhaften Haarverformung zur Verfügung zu stellen, das während der Behandlungsdauer nicht von den Haaren abläuft, eine gute Konditionierung der Haare ermöglicht, mit Wasser leicht ausspülbar ist und ein gutes Verformungsergebnis gewährleistet.

Überraschenderweise wurde nunmehr gefunden, daß die gestellte Aufgabe in hervorragender Weise gelöst wird, wenn bei der dauerhaften Haarverformung für die Durchführung der oxidativen Nachbehandlung ein Fixiermittel verwendet wird, das eine Kombination aus einem Oxidationsmittel, einem kationischen Polymer, einer amphoteren oberflächenaktiven Verbindung sowie einer nichtionogenen oberflächenaktiven Verbindung enthält.

Gegenstand der vorliegenden Erfindung ist daher ein flüssiges, leicht angedicktes Mittel zur Durchführung der oxidativen Nachbehandlung bei der dauerhaften Haarverformung ("Fixiermittel"), enthaltend (a) ein Oxidationsmittel, (b) ein kationisches Polymer, (c) eine amphotere oberflächenaktive Verbindung sowie (d) eine nichtionogene oberflächenaktive Verbindung, welches dadurch gekennzeichnet ist, daß das Gewichtsverhältnis

(I) von nichtionogener oberflächenaktiven Verbindung (d) zu amphoterer oberflächenaktiven Verbindung (c) gleich 1 : 0,3 bis 1 : 1,5, vorzugsweise 1 : 0,4 bis 1 : 0,6, ist,

(II) von nichtionogener oberflächenaktiven Verbindung (d) zu kationischem Polymer (b) gleich 1 : 0,02 bis 1 : 0,5, vorzugsweise 1 : 0,02 bis 1 : 0,1, ist und

(III) von amphoterer oberflächenaktiver Verbindung (c) zu kationischem Polymer (b) gleich 1 : 0,1 bis 1 : 1, vorzugsweise 1 : 0,15 bis 1 : 0,6, ist.

Die Vorteile dieses Fixiermittels bestehen,neben der infolge der Viskositätserhöhung unproblematischeren Applikation, insbesondere in einer deutlichen Verbesserung von Griff und Kämmbarkeit des dauerverformten Haares.

Als nichtionogene oberflächenaktive Verbindungen werden insbesondere Fettalkoholpolyglykoether mit niedrigem Ethoxylierungsgrad (beispielsweise mit 1 bis 5 Ethylenoxideinheiten im Molekül), oxethylierte Alkylphenole oder oxethylierte Sorbitanfettsäureester verwendet. Die nichtionogenen oberflächenaktiven Verbindungen sind in dem erfindungsgemäßen Fixiermittel vorzugsweise in einer Gesamtmenge von 0,1 bis 6 Gewichtsprozent enthalten.

Das Fixiermittel enthält das kationische Polymer vorzugsweise in einer Menge von 0,1 bis 2 Gewichtsprozent, während die amphotere oberflächenaktive Verbindung vorzugsweise in einer Menge von 0,1 bis 3 Gewichtsprozent enthalten ist.

Für die Verwendung in dem erfindungsgemäßen Fixiermittel sind beispielsweise die folgenden kationischen Polymere oder Mischungen dieser kationischen Polymere geeignet: Kationische Cellulosederivate, wie zum Beispiel kationische Celluloseether (beispielsweise CTFA-Polyquaternium-10), Polydimethylaminoethylmethacrylat (zu 75 % mit Dimethylsulfat oder zu 100 % mit Methylchlorid oder Methylbromid quaternisiert), Polydiallyldimethylammoniumchlorid (CTFA-Polyquaterum-6), Diallyldimethylammoniumchlorid/Hydroxyethylcellulose-Copolymere (CTFA-Polyquaternium-4), beta-Methacryloxyethyltrimethylammoniummethosulfat-Homopolymere (CTFA-Polyquaternium-14), beta-Methacryloxyethyltrimethylammoniummethosulfat/Acrylamid-Copolymere (CTFA-Polyquaternium-5), beta-Methacryloxyethyl-trimethylammoniummethosulfat/Vinylpyrrolidon-Copolymere (CTFA-Polyquaternium-11), Diallyldimethylammoniumchlorid/Acrylamid-Copolymere (CTFA-Polyquaternium-7), N-Vinylpyrrolidon/ Methacrylamidopropyltrimethylammoniumchlorid-Copolymere und kationische Chitosanderivate.

Unter diesen kationischen Polymeren sind die folgenden Verbindungen besonders bevorzugt: Polydiallyldimethylammoniumchorid, Polydimethylaminoethylmethacrylat (zu 75 % mit Dimethylsulfat quaternisiert), N-Vinylpyrrolidon/Methacrylamidopropyltrimethylammoniumchlorid-Coplymere, beta-Methacryloxyethyl-trimethylammoniumethosulfat/Vinylpyrrolidon-Copolymere und beta-Methacryloxyethyltrimethylammoniummethosulfat-Homopolymere.

Als geeignete amphotere oberflächenaktive Verbindung sind insbesondere Carboxylderivate des Imidazols, beispielsweise CTFA-Cocoamphoglycinate, CTFA-Cocoamphopropionate, CTFA-Cocoamphodiacetate oder CTFA-Cocoamphodipropionate, N-Alkylbetaine und N-Alkylamidobetaine, N-Alkylsulfobetaine, N-Alkylaminopropionate, Alkyldimethylcarboxymethylammoniumsalze mit 12 bis 18 Kohlenstoffatomen sowie Fettsäurealkylamidobetaine, beispielsweise Fettsäureamidopropyldimethylaminoessigsäurebetain, zu nennen.

Als Oxidationsmittel kann jede beliebige, bisher in Fixiermitteln verwendete oxidierende Verbindung eingesetzt werden. Beispiel für derartige oxidierende Verbindungen sind Alkalibromate, wie zum Beispiel Kalium- und Natriumbromat oder Wasserstoffperoxid. Die in dem Fixiermittel enthaltene Menge des Oxidationsmittels ist in Abhängigkeit von der Anwendungsdauer und Anwendungstemperatur unterschiedlich. Üblicherweise ist das Oxidationsmittel in einer Menge von etwa 0,1 bis 15 Gewichtsprozent enthalten.

Der pH-Wert der gebrauchsfertigen wäßrigen Lösung des Fixiermittels beträgt in Abhängigkeit vom verwendeten Oxidationsmittel etwa 1,5 bis 8,5. Bei Verwendung von Wasserstoffperoxid als Oxidationsmittel beträgt der pH-Wert vorzugsweise 1,5 bis 6, während bei Verwendung von Alkalibromaten ein pH-Wert von 6 bis 8,5 bevorzugt ist.

Selbstverständlich kann das Fixiermittel alle für derartige Mittel üblichen Zusatzstoffe, wie Farbstoff, Trübungsmittel oder Alkohole, wie beispielsweise Ethanol, Propanol, Isopropanol und 1,2-Propylenglykol , Lösungsvermittler, Puffersubstanzen, Parfümöle sowie haarkonditionierende oder haarpflegende Bestandteile, wie zum Beispiel Lanolinderivate, Cholesterin oder Betain, enthalten.

Weiterhin können dem Fixiermittel weitere Zusatzstoffe, beispielsweise Quell- und Penetrationstoffe, wie zum Beispiel Harnstoff, 2-Pyrrolidon, 1-Methyl-2-pyrrolidon und Dipropylenglykolmonomethylether, sowie Peroxidstabilisatoren, beispielsweise aromatische Sulfonsäuren, ortho-Phosphorsäure, Poly- oder Pyrophosphorsäuren, saure Salze starker Säuren, organische Sulfonsäuren, ortho-Phosphorsäure, Poly-oder Pyrophosphorsäuren, saure Salze starker Säuren, organische Säuren, Phenacetin, Phenol, Thymol oder alpha-Bisabolol, zugesetzt werden.

Das erfindungsgemäße Mittel besitzt zweckmäßigerweise eine Viskosität von 2,5 bis 50 mPa•s, wobei eine Viskosität von 5 bis 30 mPa•s besonders bevorzugt ist.

Vorzugsweise liegt das erfindungsgemäße Mittel zur Durchführung der oxidativen Nachbehandlung bei der dauerhaften Haarverformung in Form eines Zweikomponentenpräparates vor und wird erst unmittelbar vor der Anwendung durch Vermischen einer homogenen, das kationische Polymer sowie die amphotere und die nichtionogene oberflächenaktive Verbindung enthaltenden Zusammensetzung (Komponente 1) mit einer wäßrigen Wasserstoffperoxidlösung (Komponente 2) hergestellt.

3

Bei Verwendung des vorstehend beschriebenen Fixiermittels für die oxidative Nachbehandlung bei der dauerhaften Haarverformung werden das Abfließen des Fixiermittels von den Haaren vermieden sowie Griff und Kämmbarkeit des Haares verbessert und gleichzeitig das Gesamtumformungsergebnis günstig beeinflußt.

Ein weiterer Gegenstand der vorliegenden Erfindung ist daher ein Verfahren zur dauerhaften Haarverformung, bei dem man das Haar bevor und/oder nachdem man es in die gewünschte Form bringt mit einem keratinreduzierenden Verformungsmittel behandelt, mit Wasser spült, sodann mit einem Fixiermittel oxidativ nachbehandelt, mit Wasser spült, anschließend zur Frisur legt und sodann trocknet, welches dadurch gekennzeichnet ist, daß für die oxidative Nachbehandlung das vorstehend beschriebene erfindungsgemäße Fixiermittel verwendet wird.

Bei dem erfindungsgemäßen Verfahren wird das Haar gewaschen, mit einem Handtuch frottiert, in einzelne Strähnen aufgeteilt und auf Wickler gewickelt. Der Durchmesser der Wickler beträgt hierbei, je nachdem ob eine Dauerwellung oder eine Entkräuselung der Haare gewünscht wird, entweder 5 bis 13 Millimeter oder etwa 15 bis 35 Millimeter. Auf das Haar wird vor und/oder nach dem Wickeln auf Wickler eine für die dauerhafte Haarverformung ausreichende Menge des Verformungsmittels aufgetragen. Die für die dauerhafte Haarverformung erforderliche Gesamtmenge des Verformungsmittels beträgt im allgemeinen etwa 80 bis 120 Gramm.

Die bei dem erfindungsgemäßen Verfahren verwendbaren Verformungsmittel enthalten übliche keratinreduzierende Verbindungen, wie zum Beispiel bestimmte Mercaptoverbindungen, insbesondere Thioglycerin, Cysteamin, Cystein sowie Salze oder Ester von Mercaptocarbonsäuren. Diese Verformungsmittel enthalten die keratinreduzierenden Verbindungen in den für derartige Mittel üblichen Mengen, beispielsweise die Ammoniumsalze der Thioglykol- oder Thiomilchsäure in einer Menge von etwa 2 bis 12 Gewichtsprozent. Der pH-Wert dieser Verformungsmittel beträgt im allgemeinen etwa 7 bis 11, wobei die Einstellung des pH-Wertes vorzugsweise mit Ammoniak, Monoethanolamin, Ammoniumcarbonat oder Ammoniumhydrogencarbonat erfolgt. Bei sauer (zum Beispiel auf pH = 6,5 bis 6,9) eingestellten Verformungsmittel werden als keratinreduzierende Verbindungen, vorzugsweise Ester von Mercaptocarbonsäuren, wie beispielsweise Monothioglykolsäureglykolester- oder -glycerinester, in einer Konzentration von etwa 2 bis 25 Gewichtsprozent verwendet.

Die Verformungsmittel können weiterhin alle für derartige Mittel üblichen Zusatzstoffe, beispielsweise Quell- und Penetrationsstoffe, Verdickungsmittel, Netzmittel und Emulgatoren, Alkohole, Lösungsvermittler, Stabilisatoren, Farbstoffe, Parfümöle sowie haarkonditionierende oder haarpflegende Bestandteile, enthalten. Die vorstehend genannten Zusatzstoffe werden in den für solche Zwecke üblichen Mengen verwendet, zum Beispiel die Netzmittel und Emulgatoren in Konzentrationen von etwa 0,2 bis 30 Gewichtsprozent, während die Verdickungsmittel in einer Menge von etwa 0,1 bis 25 Gewichtsprozent in dem Verformungsmittel enthalten sein können.

Das bei dem erfindungsgemäßen Verfahren verwendete Verformungsmittel kann sowohl in Form einer wäßrigen Lösung oder Emulsion als auch in verdickter Form auf wäßriger Basis, insbesondere als Creme, Gel oder Paste, oder in Form eines Aerosolschaums vorliegen.

Nach einer für die dauerhafte Haarverformung ausreichenden Einwirkungszeit, welche je nach der Haarbeschaffenheit, dem pH-Wert und der Verformungswirksamkeit des Verformungsmittels sowie in Abhängigkeit von der Anwendungstemperatur etwa 5 bis 45 Minuten (5 bis 20 Minuten mit Wärmeeinwirkung; 20 bis 45 Minuten ohne Wärmeeinwirkung) beträgt, wird das Haar mit Wasser gespült und sodann mit etwa 80 bis 120 Gramm des vorstehend beschriebenen erfindungsgemäßen Fixiermittels oxidativ nachbehandelt.

Nach einer Einwirkungszeit von etwa 1 bis 20 Minuten werden die Wickler entfernt und das abgewickelte Haar, falls erforderlich, nochmals mit dem Fixiermittel oxidativ nachbehandelt. Sodann wird das Haar mit Wasser gespült, zur Frisur gelegt und getrocknet.

Das so behandelte Haar besitzt eine gleichmäßige und haltbare Umformung und ist hervorragend konditioniert.

Die nachfolgenden Beispiele dienen der näheren Erläuterung des Gegenstandes der vorliegenden Erfindung.

**Beispiele**

**Beispiel 1 Fixiermittel auf Wasserstoffperoxidbasis**

| Komponente 1 | |
|---|---|
| 20,0 g | Polyoxyethylen(3)-laurylether |
| 9,0 g | Kokosdimethylglycin |
| 1,8 g | Poly(dimethylaminoethylmethacrylat), zu 75 % quaternisiert mit Dimethylsulfat |
| 1,2 g | Cocoamphoglycinate |
| 5,0 g | Parfümöl |
| 33,0 g | Wasser, vollentsalzt |
| 30,0 g | 1,2-Propylenglykol |
| 100,0 g | |

| Komponente 2 | |
|---|---|
| 3,25 g | Wasserstoffperoxid |
| 0,07 g | N-Acetyl-p-aminophenol |
| 0,05 g | o-Phosphorsäure |
| 96,63 g | Wasser, vollentsalzt |
| 100,00 g | |

15 g der Komponente 1 und 85 g der Komponente 2 werden unmittelbar vor der Anwendung miteinander vermischt. Das so erhaltene gebrauchsfertige Mittel besitzt einen pH-Wert von 4,8. Die Viskosität des Mittels beträgt 5,5 mPa•s.

Im Anschluß an die Behandlung der Haare mit einem Verformungsmittel auf Thioglykolatbasis (10-prozentige Ammoniumthioglykolatlösung; pH-Wert der Thioglykolatlösung: 9,0; Einwirkungszeit: 20 Minuten bei Raumtemperatur) werden die Haare mit Wasser gespült, und sodann 85 g des vorstehend beschriebenen Fixiermittels mit einem Schwamm aufgetragen. Nach einer Einwirkungszeit von 10 Minuten werden die Wickler entfernt und das Haar mit lauwarmen Wasser ausgespült. Anschließend wird das Haar zur Frisur gelegt und sodann getrocknet. Das so behandelte Haar besitzt eine gleichmäßige Umformung und einen weichen Griff.

**Beispiel 2 Fixiermittel auf Wasserstoffperoxidbasis**

| Komponente 1 | |
|---|---|
| 20,0 g | Polyoxethylen(4)-laurylether |
| 9,0 g | Kokosdimethylglycin |
| 1,8 g | quaternäres Copolymerisat aus Vinylpyrrolidon und Methacrylamidoprpyltrimethylammoniumchlorid |
| 1,2 g | Cocoamphoglycinate |
| 5,0 g | Parfümöl |
| 36,0 g | 1,2-Propylenglykol |
| 27,0 g | Wasser, vollentsalzt |
| 100,0 g | |

| Komponente 2 | |
|---|---|
| 2,75 g | Wasserstoffperoxid |
| 0,50 g | Dinatriumhydrogenphosphat x 12 $H_2O$ |
| 0,15 g | Phosphorsäure |
| 0,07 g | N-Acetyl-p-aminophenol |
| 96,53 g | Wasser, vollentsalzt |
| 100,00 g | |

Unmittelbar vor der Anwendung wird durch Vermischen von 7 g der Komponente 1 und 93 g der Komponente 2 das gebrauchsfertige Fixiermittel (pH = 3,9; Viskosität = 6,5 mPa•s) hergestellt.

Die Haare werden in der in Beispiel 1 beschriebenen Weise behandelt, wobei jedoch an Stelle des thioglykolathaltigen Verformungsmittels ein Verformungsmittel auf Thioglykolsäureester-Basis (pH = 6,5; Thioglykolsäuremonoglycerinsäureester-Gehalt = 20 Gewichtsprozent; Einwirkungsdauer: 20 Minuten) verwendet wird und die Einwirkungszeit des Fixiermittels 8 Minuten beträgt.

**Beispiel 3 Fixiermittel auf Bromatbasis**

| Komponente 1 | |
|---|---|
| 25,0 g | Polyoxethylen(3)-laurylether |
| 9,0 g | Kokosdimethylglycin |
| 0,6 g | Poly(dimethylaminoehtylmethacrylat), zu 75 % mit Dimethylsulfat quaternisiert |
| 1,2 g | Cocoamphoglycinate |
| 5,0 g | Parfümöl |
| 31,2 g | Wasser, vollentsalzt |
| 28,0 g | 1,2-Propylenglykol |
| 100,0 g | |

| Komponente 2 | |
|---|---|
| 12,0 g | Natriumbromat |
| 1,0 g | Dinatriumhydrogenphosphat X 12 $H_2O$ |
| 0,7 g | Natriumhydrogenphosphat x 2 $H_2O$ |
| 86,3 g | Wasser, vollentsalzt |
| 100,0 g | |

Unmittelbar vor Gebrauch werden 20 g der Komponente 1 und 80 g der Komponente 2 zu einem gebrauchsfertigen Fixiermittel mit einem pH-Wert von 6,2 und einer Viskosität von 24,4 mPa•s vermischt.

Die Behandlung der zu verformenden Haare erfolgt in der in Beispiel 1 angegebenen Weise; als Reduktionsmittel wird jedoch eine 8-prozentige Thiomilchsäurelösung (pH = 9,5; Einwirkungszeit: 15 Minuten bei 35° C) verwendet. Die Einwirkungsdauer des Fixiermittels beträgt 15 Minuten.

Alle in der vorliegenden Anmeldung enthaltenen Prozentangaben stellen - soweit nicht anders angegeben - Gewichtsprozent dar. Die Viskositäten wurden mit Hilfe einer Viskowaage der Firma Haake bei einer Meßtemperatur von 30 Grad Celsius unter Verwendung des Stabes 1 bei einem Auflagegewicht von 5 g bestimmt.

**Patentansprüche**

1. Flüssiges, leicht angedicktes Mittel zur Durchführung der oxidativen Nachbehandlung bei der dauerhaften Haarverformung, enthaltend
   (a) ein Oxidationsmittel,
   (b) ein kationisches Polymer,
   (c) eine amphotere oberflächenaktive Verbindung und

(d) eine nichtionogene oberflächenaktive Verbindung,
dadurch gekennzeichnet, daß das Gewichtsverhältnis

(I) von (d):(c) gleich 1:0,3 bis 1:1,5 ist,

(II) von (d):(b) gleich 1:0,02 bis 1:0,5 ist und

(III) von (c):(b) gleich 1:0,1 bis 1:1 ist.

2. Mittel nach Anspruch 1, dadurch gekennzeichnet, daß es einen pH-Wert von 1,5 bis 8,5 besitzt.

3. Mittel nach Anspruch 1 oder 2, dadurch gekennzeichnet, daß das Oxidationsmittel ausgewählt ist aus Alkalibromaten und Wasserstoffperoxid.

4. Mittel nach einem der Ansprüche 1 bis 3, dadurch gekennzeichnet, daß das Oxidationsmittel in einer Menge von 0,1 bis 15 Gewichtsprozent enthalten ist.

5. Mittel nach einem der Ansprüche 1 bis 4, dadurch gekennzeichnet, daß das kationische Polymer ausgewählt ist aus kationischen Cellulosederivaten, Polydimethylaminoethylmethacrylat (zu 75 % mit Dimethylsulfat oder zu 100 % mit Methylchlorid oder Methylbromid quaternisiert), Polydiallyldimethylammoniumchlorid, Diallyldimethylammoniumchlorid/Hydroxyethylcellulose-Copolymeren, beta-Methacryloxyethyltrimethylammoniummethosulfat/Acrylamid-Copolymeren, beta-Methacryloxyethyltrimethylammoniummethosulfat/Vinylpyrrolidon-Copolymeren, beta-Methacryloxyethyltrimethylammoniummethosulfat-Homopolymeren, Diallyldimethylammoniumchlorid/Acrylamid-Copolymeren, N-Vinylpyrrolidon/Methacrylamidopropyldimethylammoniumchlorid-Copolymeren und kationischen Chitosanderivaten.

6. Mittel nach einem der Ansprüche 1 bis 5, dadurch gekennzeichnet, daß das kationische Polymer in einer Menge von 0,1 bis 2 Gewichtsprozent enthalten ist.

7. Mittel nach einem der Ansprüche 1 bis 6, dadurch gekennzeichnet, daß die amphotere oberflächenaktive Verbindung ausgewählt ist aus Carboxylderivaten des Imidazols, N-Alkylbetainen, N-Alkylamidobetainen, N-Alkylsulfobetainen, N-Alkylaminopropionaten, Alkyldimethylcarboxymethylammoniumsalzen mit 12 bis 18 Kohlenstoffatomen und Fettsäurealkylamidobetainen.

8. Mittel nach einem der Ansprüche 1 bis 7, dadurch gekennzeichnet, daß die amphotere oberflächenaktive Verbindung in einer Menge von 0,1 bis 3 Gewichtsprozent enthalten ist.

9. Mittel nach einem der Ansprüche 1 bis 8, dadurch gekennzeichnet, daß die nichtionogene oberflächenaktive Verbindung ausgewählt ist aus Fettalkoholpolyglykolethern mit 1 bis 5 Ethylenoxideinheiten im Molekül, oxethylierten Alkylphenolen und oxethylierten Sorbitanfettsäureestern.

10. Mittel nach einem der Ansprüche 1 bis 9, dadurch gekennzeichnet, daß die nichtionogene oberflächenaktive Verbindung in einer Menge von 0,1 bis 6 Gewichtsprozent enthalten ist.

11. Mittel nach einem der Ansprüche 1 bis 10, dadurch gekennzeichnet, daß es in Form eines 2-Komponenten-Präparates vorliegt und unmittelbar vor der Anwendung durch Vermischen einer homogenen, das kationische Polymer sowie die amphotere und nichtionogene oberflächenaktive Verbindung enthaltenden Zusammensetzung (Komponente 1) mit einer wäßrigen Wasserstoffperoxidlösung (Komponente 2) hergestellt wird.

12. Verfahren zur dauerhaften Haarverformung, bei dem man das Haar bevor und/oder nachdem man es in die gewünschte Form bringt mit einem keratinreduzierenden Verformungsmittel behandelt, mit Wasser spült, anschließend zur Frisur legt und sodann trocknet, dadurch gekennzeichnet, daß für die oxidative Nachbehandlung ein Mittel nach einem der Ansprüche 1 bis 11 verwendet wird.

**Claims**

1. Slightly thickened, fluid agent for carrying out the oxidative after-treatment during the permanent shaping of hair, comprising:

(a) an oxidising agent;

(b) a cationic polymer;

(c) an amphoteric surfactant compound; and

(d) a non-ionic surfactant compound

characterised in that the proportion by weight

(I) of (d):(c) is from 1:0.3 to 1:1.5;

(II) of (d):(b) is from 1:0.02 to 1:0.5; and

(III) of (c):(b) is from 1:0.1 to 1:1.

2. Agent according to Claim 1, characterised in that it has a pH value of 1.5 to 8.5.

3. Agent according to Claim 1 or Claim 2, characterised in that the oxidising agent is selected from alkalimetal bromates and hydrogen peroxide.

4. Agent according to any one of Claims 1 to 3, characterised in that it includes a quantity of from 0.1 to 15 % by weight of the oxidising agent.

5. Agent according to any one of Claims 1 to 4, characterised in that the cationic polymer is selected from cationic cellulose derivatives, polydimethylaminoethyl methacrylate (quaternised to 75% with dimethyl sulphate or to 100% with methyl chloride or methyl bromide), polydiallyldimethylammonium chloride, diallyldimethylammonium chloride/hydroxyethylcellulose copolymers, beta-methacryloxyethyl-trimethylammonium methosulphate/acrylamide copolymers, beta-methacryloxyethyltrimethylammonium methosulphate/vinylpyrrolidone copolymers, beta-methacryloxyethyl-trimethylammonium methosulphate homopolymers, diallyldimethylammonium chloride/acrylamide copolymers, N-vinylpyr-rolidone/methacrylamidopropyldimethylammonium chloride copolymers and cationic chitosan derivatives.

6. Agent according to any one of Claims 1 to 5, characterised in that it comprises a quantity of from 0.1 to 2 % by weight of the cationic polymer.

7. Agent according to any one of Claims 1 to 6, characterised in that the amphoteric surfactant compound is selected from carboxyl derivatives of the imidazoles, N-alkylbetaines, N-alkylamidobetaines, N-alkylsulphobetaines, N-alkylaminopropionates, alkyldimethylcarboxymethylammonium salts with 12 to 18 carbon atoms and fatty-acid alkylamidobetaines.

8. Agent according to any one of Claims 1 to 7, characterised in that it comprises a quantity of from 0.1 to 3 % by weight of the amphoteric surfactant compound.

9. Agent according to any one of Claims 1 to 8, characterised in that the non-ionic surfactant compound is selected from fatty-alcohol polyglycol ethers with 1 to 5 ethylene oxide units per molecule, ethoxylated alkyl phenols and ethoxylated sorbitane esters of fatty acids.

10. Agent according to any one of Claims 1 to 9, characterised in that it comprises a quantity of from 0.1 to 6 % by weight of the non-ionic surfactant compound.

11. Agent according to any one of Claims 1 to 10, characterised in that it is available in the form of a 2-component preparation and is prepared immediately before use by the mixing of a homogeneous composition comprising the cationic polymer and the amphoteric and non-ionic surfactant compounds (component 1) with an aqueous solution of hydrogen peroxide (component 2).

12. Process for the permanent shaping of hair, in which the hair is treated with a keratin-reducing shaping agent before and/or after it is shaped as desired, is rinsed with water, is subsequently styled and is then dried, characterised in that an agent according to any one of Claims 1 to 11 is used for the oxidative after treatment.

**Revendications**

1. Agent liquide légèrement épaissi pour le posttraitement oxydant lors d'une mise en pli permanente, contenant:

(a) un agent d'oxydation,

8

(b) un polymère cationique,

(c) un composé tensioactif amphotère,

(d) un composé tensioactif non-ionogène,

caractérisé en ce que le rapport massique de

(I) (d) : (c) va de 1 : 0,3 à 1 : 1,5,

(II) (d) : (b) va de 1 : 0,02 à 1 : 0,5, et

(III) (c) : (b) va de 1 : 0,1 à 1 : 1.

2. Agent selon la revendication 1, caractérisé en ce qu'il possède une valeur du pH allant de 1,5 à 8,5.

3. Agent selon la revendication 1 ou 2, caractérisé en ce que l'agent d'oxydation est choisi parmi les bromates alcalins et le peroxyde d'hydrogène.

4. Agent selon l'une des revendications 1 à 3, caractérisé en ce que l'agent d'oxydation est contenu à raison de 0,1 à 15% en poids.

5. Agent selon l'une des revendications 1 à 4, caractérisé en ce que le polymère cationique est choisi parmi les dérivés cationiques de la cellulose, le méthacrylate de polydiméthylaminoéthyle (quaternisé à 75% avec du sulfate de diméthyle, ou à 100% avec du chlorure de méthyle ou du bromure de méthyle), du chlorure de polydiallylediméthylammonium, des copolymères de diallyldiméthylammonium/hydroxyéthylcellulose, des copolymères de méthosulfate de $\beta$-méthacryloxyéthyl triméthylammonium et d'acrylamide, des copolymères de méthosulfate de $\beta$-méthacryloxyéthyltriméthylammonium et de vinylpyrrolidone, d'homopolymères de méthosulfate de $\beta$-métacryloxyéthyltriméthylammonium, des copolymères de chlorure de diallyldiméthylammonium et d'acrylamide, des copolymères de N-vinylpyrrolidone et de chlorure de méthacrylamidopropyldiméthylammonium, et des dérivés cathionique du chitosane.

6. Agent selon l'une des revendications 1 à 5, caractérisé en ce que le polymère cationique est présent à raison de 0,1 à 2% en poids.

7. Agent selon l'une des revendications 1 à 6, caractérisé en ce que l'agent tensioactif amphotère est choisi parmi les dérivés carboxylés de l'imidazol, des N-alkylbétaïnes, des N-alkyl-amidobétaïnes, des N-alkylsulfobétaïnes, des N-alkyle-aminopropionates, des sels d'alkyldiméthyl-carboxyméthylammonium possédant de 12 à 18 atomes de carbone, et alkylamidobétaïnes d'acides gras.

8. Agent selon l'une des revendications 1 à 7, caractérisé en ce que le composé tensioactif amphotère est présent à raison de 0,1 à 3% en poids.

9. Agent selon l'une des revendications 1 à 8, caractérisé en ce que le composé tensioactif non-ionogène est choisi parmi les éthers de polyglycol et d'alcools gras avec 1 à 5 motifs d'oxyde d'éthylène dans la molécule, les alkylphénols oxyéthylés, et les esters de sorbitane d'acides gras oxyéthylés.

10. Agent sel on l'une des revendications 1 à 9, caractérisé en ce que le composé tensioactif non-ionogène est présent à raison de 0,1 à 6% en poids.

11. Agent selon l'une des revendications 1 à 10, caractérisé en ce qu'il se présente sous la forme d'une préparation à deux composants, et immédiatement avant l'utilisation, on prépare par mélangeage une composition homogène contenant un polymère cationique homogène et le composé amphotère ainsi que le composé tensioactif non-ionogène (composant 1) avec une solution aqueuse de peroxyde d'hydrogène (composant 2).

12. Procédé de mise en pli permanente de cheveux, selon lequel les cheveux sont traités avant et/ou après leur mise sous la forme désirée avec un agent de mise en forme réducteur de la kératine, rincés à l'eau, puis coiffés et ensuite séchés, caractérisé en ce que pour le post-traitement oxydant on utilise un agent selon l'une des revendications 1 à 11.